# EUROPEAN PATENT APPLICATION

(11) **EP 3 925 581 A1**
(43) Date of publication of application: **22.12.2021**
(21) Application number: 21386035.6
(22) Date of filing: 15.06.2021
(51) Int. Cl.: A61F 9/00

(54) **DEVICE FOR THE SAFE AND EASY INSTILLATION OF EYE DROPS**

(30) Priority: 18.06.2020 GR 20200100346
(71) Applicant: Oanta, Mariana, 11471 Athens (GR)
(72) Inventor: Oanta, Mariana, 11471 Athens (GR)

(57) **Abstract**

The innovative device for the safe and easy instillation of eye drops consists of two independent pieces , the reclosable container **(10)** with the safety cap **(15)** and the structural support **(20),** consisting of three parts, the nasal support base **(23),** the vertical boom arm **(24)** and the reception space **(27)** of the innovative reclosable container **(10).**

The reclosable container **(10)** has the safety cap **(15),** which protects the content of the container against contamination during its reuse and can be securely supported with the appropriately shaped flat projections **(11, 11')** in the reception space **(27)** of the structural support **(20).**

The structural support **(20)** after being provided with the reclosable container **(10)** rests on the patient's nose and with very easy handling the pharmaceutical solution is accurately instilled into the eye or eyes of the patient. In this way the patients becomes self-sufficient and carries out the prescribed treatment without the assistance of a third party.

## Description

### TECHNICAL FIELD

The invention refers to an innovative device which consists of an innovative reclosable container with a safety cap that fits into a recessed slot of one innovative structural support for the safe and easy instillation of eye drops.

### BACKGROUND OF THE INVENTION

Up to date various containers are known in the pharmaceutical market, which do not fully guarantee the sterile conditions of the active pharmacological substances stored within them after their first opening and do not offer any facilitations to the patients for the proper application of a prescribed treatment. More specifically, due to the incomplete planning of the containers, they often cause injuries to the surface of the eye and in the periocular area, it their caps may be lost after their first opening, consequently infecting their contents and be rejected without the rest of the contents left being reusable anymore, which means a financial burden for the patients. Beyond that, every container, currently available on the pharmaceutical market contains usually a larger quantity of drug solution than required for the therapy for example one drop of instillation and in fact, it is indicated on the instructions that after its opening and the first use of the container, this must be discarded. So, the largest amount of the drug is not used for the therapy and of course this is a great financial waste and burden for the patients, for the social security organisation and a significant environmental pollution, in which the discarded container finally ends up. In order to apply a prescribed treatment with the existing containers, as it provided on their indications that the patient with the one hand can slightly pull down the lower eyelid and with the other hand, after bearing the open spout of the container at a certain distance above the surface of the eye, to instill into it one or more prescribed drops by squeezing the walls of the container. This seemingly simple instillation process is often performed incorrectly by the patients and the instillations do not reach the surface of the eye, but the surrounding area or cannot be performed by themselves and thus they need help from someone close to them, who either cannot be found there or even if this assistant is found, they may be not available at the times the instillation is specified. At the same time there may be on the market different holders for these containers , which these holders are used for a general purpose without being specifically designed for a particular container and on the one hand, they do not keep them in a safe position, and on the other hand, their positioning is very close to the ocular surface, that there is a risk of injury by them at the slightest wrong maneuver of use.

So, the absence of a device, which can be activated with very simple manipulations, to guarantee the safe instillation of the eye drops and making patients self-sufficient, led us to research, study and design one innovative structural support and one innovative reclosable container with a safety cap, adapted to the above structural support, which together function as a coupling connection, type male/ female system.

The advantages of this invention are:
a) In the innovative reclosable container with a safety cap, due to the fact that the cap is securely locked even after its first opening, the containers continue to keep its contents sterile until the whole active pharmacological substance is consumed and in this way, the therapy of the patient is safer, more efficient and more economical.
b) In this innovative reclosable container with a safety cap, in addition to all the new changes it is proposed to have a permanent printing of the pharmaceutical information, for example the name of the pharmacological substance, the expiration date, its casing can be manufactured by a non-toxic material and in addition, to having thermal insulation and environmentally friendly properties to protect the content, on the one hand from temperatures higher than the permissible specified temperatures and on the other hand for the protection of the environment after it is discarded.
c) The innovative reclosable containers with a safety cap, either during their manufacturing, or right after it and exactly before their use, it is suggested to be marked according to the content with different distinctive colors, so as not to confuse the containers and consequently their different pharmaceutical substances.
d) At the innovative structural support , consisting of one nasal support base, vertical boom arm and reception space, the innovative reclosable container with safety cap is adjusted within a recessed slot, in an inclined position, so its content can be fully used.
e) The innovative structural support has in the upper extremity one recessed slot for the safe adaptation of the innovative reclosable container with the safety cap, so that its spout is securely positioned above the eye and at a sufficient distance to avoid any contact with it, in order not to have unwanted injuries on the surface of the eye and contaminations of the remaining content of the container.
f) The innovative structural support ensures a stable and safe distance from the surface of the eye and offers a simple maneuver for the programmed instillation.
g) The innovative structural support is suggested to be manufactured from a low value and high strength materials, be reusable, easy to be cleaned after every use if is required and in this way the innovative device for the safe and easy instillation of the eye drops as a whole to be affordable to the patients who need it.
h) The innovative structural support can be rotated clockwise or counterclockwise around its longitudinal axis to approach both eyes and to be able to perform instillations in one or both eyes alternately.

As it is well known, for the therapy of ophthalmic diseases we often need instillation of eye drops from one or alternately from several drugs. The current needs in the ophthalmic treatment require the most frequent use of pharmaceutical solutions without preservatives as not to aggravate the surface of the eye with toxic substances and for this reason we need to use the reclosable containers safely in limited dosages.

### DISCLOSURE OF THE INVENTION

Starting from the existing containers which currently are available in the pharmaceutical market, which do not meet the above mentioned needs because they do not have the advantageous properties of the present innovation, according to which it is proposed to be manufactured one consumable, removable innovative reclosable container with a safety cap, which is placed in the recessed slot of an innovative structural support at the same time and both of these parts work as a whole, a type of a coupling connection (male/famele), offering practical and cost-effective solutions for the patients with ophthalmic diseases. The innovative reclosable container with the safety cap keeps its contents sterile even after its first opening until all the active pharmacological substance is consumed. There is a possibility for the reclosable containers with a safety cup to be marked either during their manufacturing or afterwards and before their immediate use, in order that different content of the containers to have a different distinctive color so that they cannot be confusing when more than one container is used alternately in the same therapy. The marking takes place in the casing of the reclosable container either during manufacture or by hand with a marker of permanent ink, which will have the same color as the color marking of its structural support.

If this innovative device is addressed to the general population, the possibility of a color marking offers additional security and assistance in the implementation of the prescribed ophthalmological therapy without the fear of incalculable complications, especially for people with poor vision, illiterate, unskilled support staff, who can easily confuse the ophthalmic containers and consequently also confuse their active pharmacological solutions.

The present innovation is a innovative device for the safe and easy instillation of eye drops, consisting of two independent parts, the innovative reclosable container with the safety cap and its innovative structural support. The innovative structural support bear at one end the reception space in which the innovative reclosable container with the safety cap can be adapted and at the other end we have the nasal support base, adapted to the patient's nose.

### BRIEF DESCRIPTION OF THE DRAWINGS

To understand better the present innovation, the figures of its individual constitutive parts are presented below:
**FIG. 1** shows a perspective view of the innovative reclosable container with the open spout.
**FIG. 2** shows a perspective view of the safety cap of the innovative reclosable container
**FIG. 3** shows a perspective view of the innovative reclosable container with the spout closed by its safety cap
**FIG.4** illustrates the safety cap of the innovative reclosable container as it is illustrated in FIG.2, but from another perspective view
**FIG.5** shows a perspective view of the variant of the safety cap with a protruding wing
**FIG.6** shows a perspective view of the section A-A of the innovative reclosable container's body as it is illustrated in FIG.1
**FIG.7** shows a perspective view of the innovative structural support of the innovative reclosable container
**FIG.8** shows a perspective view of the section B-B of the vertical boom arm as it is illustrated in FIG.7 together with the nasal support base, saddle shaped
**FIG. 9** is a frontal view of the section B-B of the vertical boom arm as it is illustrated in FIG.7 together with the nasal support base, which is fitted in its inner surface with a lining of a soft material
**FIG.10** shows a perspective view of the section C-C of the vertical boom arm as it is illustrated in FIG.7 together with the reception space
**FIG.11** shows a perspective view of the section C-C of the vertical boom arm as it is illustrated in FIG.7 together with the shaped reception space and with the reclosable container in a position that can be inserted into this space
**FIG.12** shows a perspective view of the locking screw of the innovative reclosable container
**FIG.13** shows a perspective view of the general image of the innovative device for a safe and easy instillation of the eye drops of the reclosable container and its structural support, which is adjusted to the upper nasal part of the patient

The above attached drawings of the reclosable container and its structural support are not limited as to dimensions, materials, the method of manufacturing or other factors and only serve here for the understanding of the invention. This provides the possibility of manufacturing many variants of the present invention.

### DETAILED DESCRIPTION OF THE INVENTION

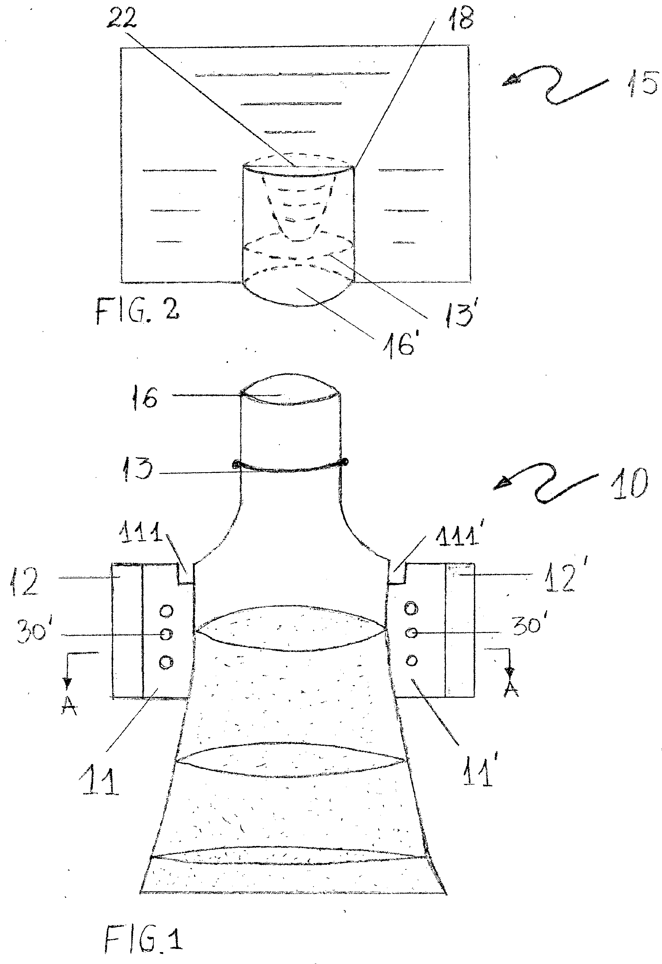

**FIG.1** illustrates in perspective view the reclosable container (10) with an open spout **(16),** shown bilaterally and at the same level with one flat projection **(11)** and (**11**') in the shape of a rectangular, bearing holes **(30')** and at their ends a cylindrical protuberance **(12)** and **(12')** is found. At the basis of this flat projection **(11)** and **(11')** and only at on one side of it, there is a notch **(111)** and **(111'),** which comes in conjunction with the locking projection **(28')** of the reception space **(27)** of the container, which is not shown in this drawing, in order that together with the cylindricals protuberances **(12, 12')** and with the terminal closed side **(28),** which again is not shown in the present illustration, to immobilize the reclosable container **(10)** in the reception space **(27).** On the outer wall of the neck of the reclosable container **(10)** and at a very short distance from its end to the spout **(16),** an annular projection **(13)** is provided.

**FIG. 2** illustrates a perspective view of the safety cap **(15).** On its inner surface of the cylindrical casing **(18)** of the head **(22)** of the safety cap **(15),** an annular groove **(13')** is provided, which is intertwined with the corresponding annular projection **(13)** of the reclosable container's **(10)** neck according to FIG.1 and in this way we can avoid an unintentional uncapping of this container and a threatened contamination of its contents. The outer diameter of the spout **(16)** of the reclosable container **(10),** which again is shown in FIG.1, is slightly less than the inside diameter of the external spout **(16')** of the cylindrical casing **(18)** of the head **(22)** of the safety cap **(15)** to bring them in contact with each other with great and constant pressure.

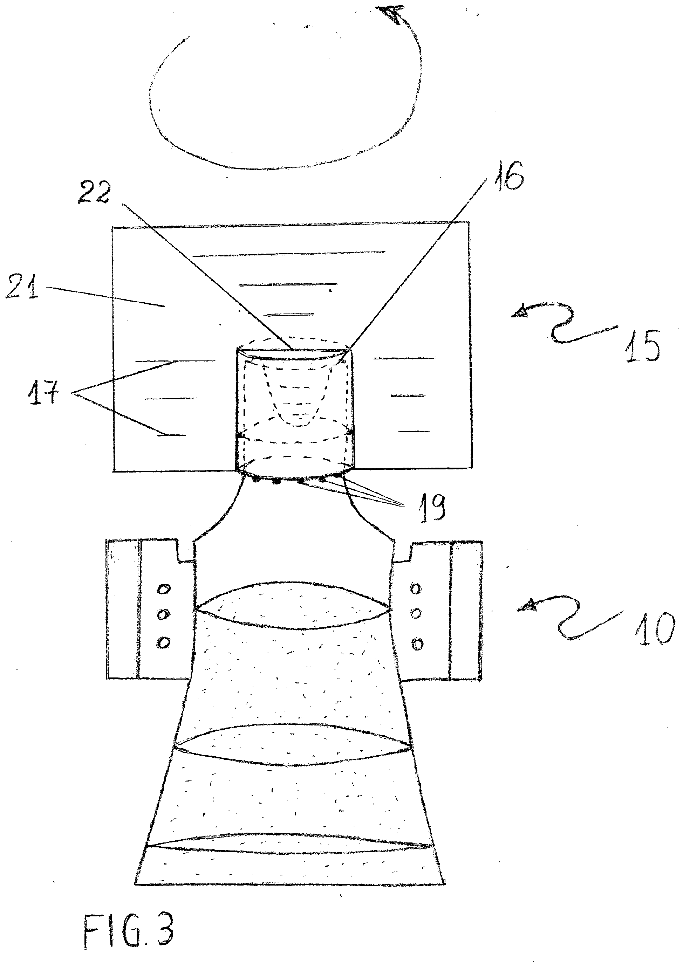

**FIG. 3** illustrates the innovative reclosable container **(10)** in a position in which the safety cap **(15)** has sealed the spout **(16)** of this container and is fixed to its walls through the sealing points **(19).** For an easy uncapping of the reclosable container **(10),** on both sides on the thin Π shaped plastic sheet **(21)** of the head **(22)** of the safety cap **(15)** are positioned linear low-height non-slip protuberances **(17),** to successfully perform the repeated movements to open and close the reclosable container **(10)** after its first opening until all its content is consumed and before the expiration of a twelve-hour period or another period of time as it is recommended by the pharmaceuticals manufacturers. The reclosable container **(10)** and the safety cap **(15)** are manufactured of a non-toxic material, plastic or another suitable material, having thermal insulation and environmentally friendly properties.

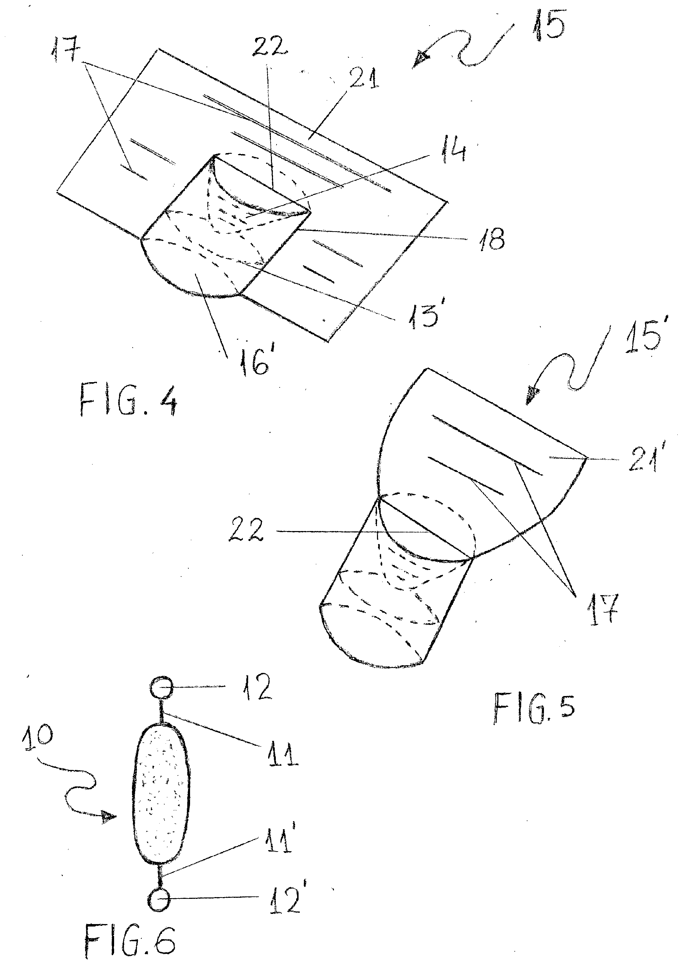

**FIG.4** illustrates another perspective view of the safety cap **(15)** composed of the head **(22)** and one thin Π shaped plastic sheet **(21)** extending to the same level towards the right, the left and above the roof of the head **(22)** and bears on both sides linear low-height non-slip protuberances **(17).** The head **(22)** of the safety cap **(15)** is consists of one cylindrical casing **(18)** in the shape of an inverted cup, which presents in its interior surface the following structural elements: a cone **(14),** projecting from the top of the cup towards the rim of the cup, an annular groove **(13')** located in its inner circular side of the cylindrical casing **(18)** between the top of the cone **(14)** and its external spout **(16').** The cone **(14),** made within the cylindrical casing **(18)** of the head **(22)** of the safety cap **(15)** comes into a conical contact with the rim of the spout **(16)** of the reclosable container **(10)** as is shown in FIG. 1 and thus ensures that the reclosures , which take place after the first opening of the container to fully achieve a liquid-tight closure while avoiding any pharmaceutical solution leakage out of the container.

**FIG.5** illustrates in perspective view the safety cap variant **(15').** This safety cap variant **(15')** is one alternative safety cap **(15)** as is shown in FIG. 4, the only difference from this, is that here the thin protruding wing **(21')** extends only above the roof of the head **(22)** and bears linear low-height non-slip protuberances **(17).**

**FIG.6** illustrates the section A-A as it is shown in FIG.1 of the body of the innovative reclosable container **(10),** showing the hollow storage space of the active pharmaceutical solution of this container with the two oppositely extending flat projections **(11)** and **(11'),** ending in two cylindricals protuberances **(12)** and **(12').** This flat projection **(11)** or **(11')** together with its cylindrical protruberance **(12)** or **(12')** are designed to be inserted in the recessed slot **(25)** of the structural support **(20),** which are not shown in this illustration, and ensure the immobilization of the reclosable container **(10).**

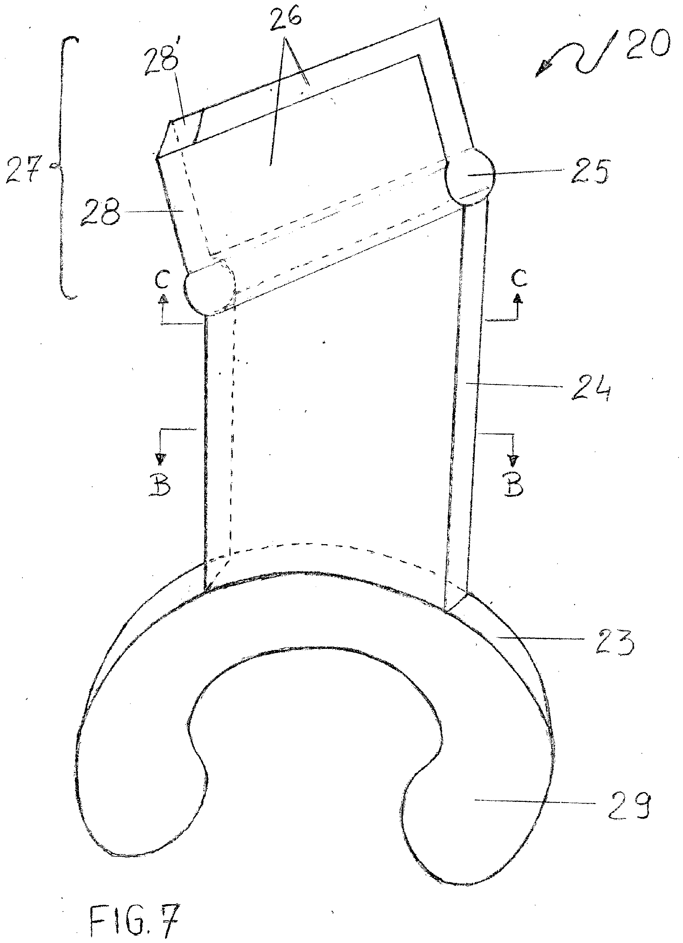

**FIG.7** illustrates the one-piece construction of the innovative structural support **(20)** of the reclosable container **(10)** which bears on one end of it the nasal support base **(23),** preferably in saddle shape, continues with the vertical boom arm **(24)** and on the other side bears the reception space **(27).** This reception space **(27)** is formed by the recessed slot **(25),** the lateral walls **(26),** the terminal closed side **(28)** with the locking projection **(28')** and into which the flat projection **(11)** or **(11')** of the reclosable container **(10)** is inserted, not shown in this drawing and is immobilised in an inclined position in relation to the horizontal level. For the immobilization of the reclosable container **(10),** the notches **(111, 111')** are provided at the bases of the flat projections **(11)** and **(11')** and at their ends the cylindricals protuberances **(12)** and **(12')** are placed in accordance with the FIG.1. This insertion is done from the top downward side of the reception space **(27)** and stops when the front part of the flat projection **(11)** or **(11')** reaches the opposite terminal closed side **(28)** and there the interlocking of the locking projection **(28')** of the reception space **(27)** with the notch **(111)** or **(111')** can be performed. The manufacturing material of the structural support **(20)** will be made of durable, rigid, easy to be cleaned, lightweight, non-toxic and environmentally friendly material e.g. plastic. On the inner surface of the saddle of the nasal support base **(23)** a lining of soft material **(29)** is placed, preferably silicone to be fitted as closely as possible to the various anatomical shapes of the patient's nose and thus offer increased stability at the structural support **(20)** to prevent it from being moved by any unintentional pushes by the patient's hands or other causes during the use.

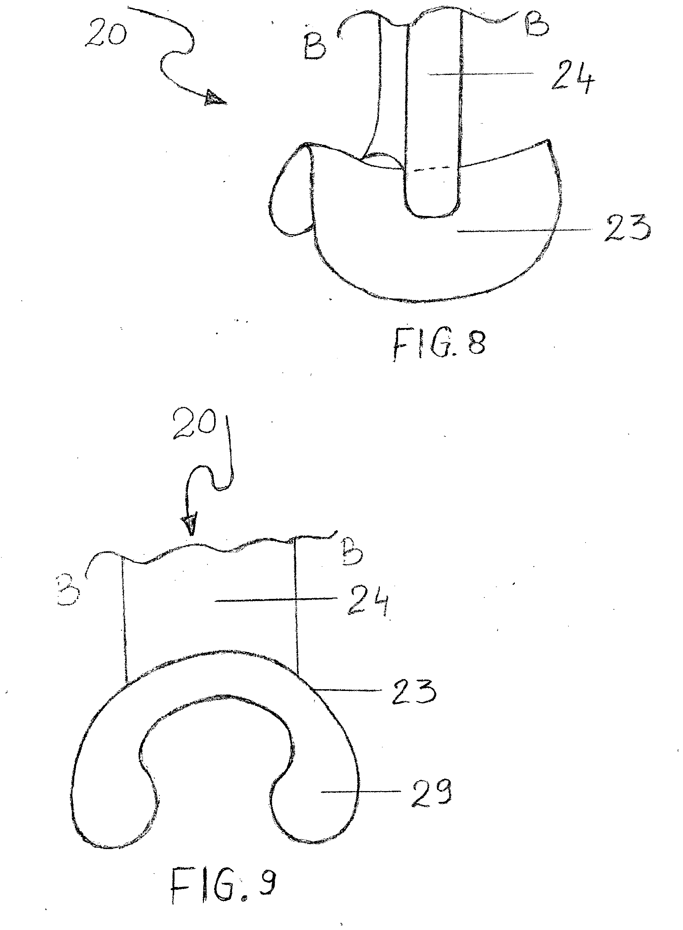

**FIG.8** illustrates the section B-B of the vertical boom arm **(24)** of the structural support **(20)** as it is shown in FIG.7 with the nasal support base **(23),** saddle shaped, where its extensive contact with the patient's nasal surface is clearly seen. This extensive contact significantly enhances the stability of the support.

**FIG. 9** illustrates the section B-B of the vertical boom arm **(24)** of the structural support **(20)** as it is shown in FIG.7 with the lining of a soft material **(29),** preferably silicone, which is placed in the inner side of the nasal support base **(23).** This nasal support base **(23),** on the one hand with the soft lining on the inner side of the saddle and on the other hand, with its extensive contact with the patient's nasal surface, it can provide vertical support of the boom arm **(24).** This design of the vertical positioning of the boom arm **(24)** on this nasal support base **(23)** has also the role of reducing the force applied by the hand which presses in gravitational direction the moment of the eye drops instillation offering even more stability and durability to the device and gives the patient more confidence to be self-sufficient in performing the required treatment.

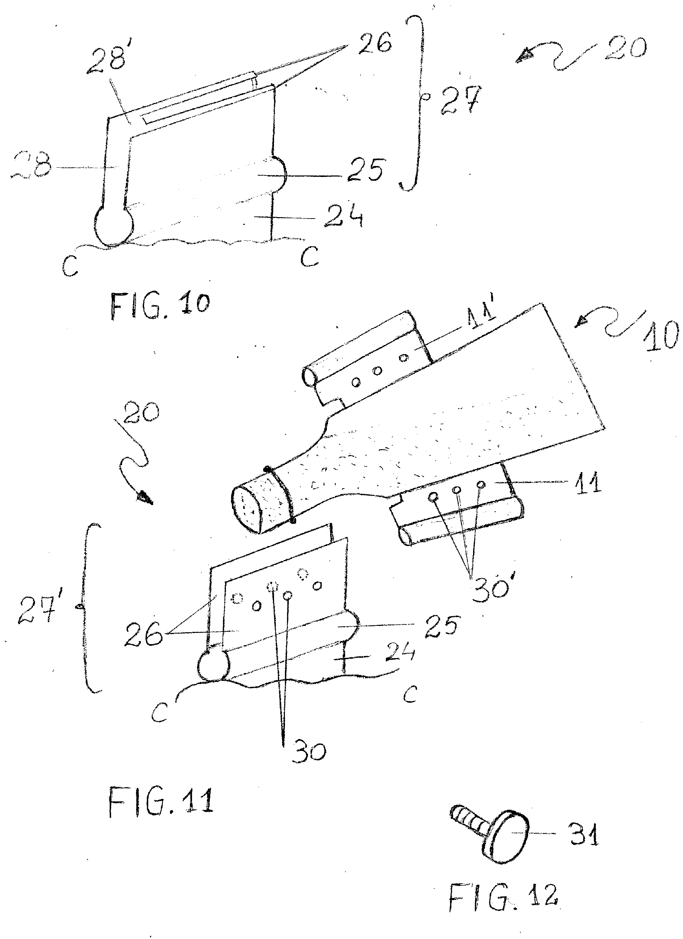

**FIG.10** illustrates the section C-C of the vertical boom arm **(24)** of the structural support **(20)** as it is shown in FIG.7 with the reception space **(27),** which consist of the recessed slot **(25)** the lateral walls **(26),** the terminal closed side **(28)** together with the locking projection **(28')** to create a tight fitting of the innovative reclosable container **(10)** in the reception space **(27)** of the structural support **(20).** This fitting holds the reclosable container **(10)** on the structural support **(20)** in an inclined position so that the contents of this container can be fully utilized.

**FIG.11** illustrates the section C-C of the vertical boom arm **(24)** of the structural support **(20)** as it is shown in FIG.7 with the modified receiving space **(27')** and with the reclosable container **(10)** in a position that can be inserted into this space. This modified reception space **(27')** is a variant of the reception space **(27)** as it is shown in FIG.7, which in this case consists of the recessed slot **(25)** and of the lateral walls **(26),** which have regulatory holes **(30)** corresponding to the holes **(30')** of the flat projection **(11)** or **(11')** of the reclosable container **(10),** which is fixed in an inclined position through of a screw or through another more convenient means of locking. The purpose of this variant is to make it possible to adjust the position of the reclosable container **(10)** inside the modified reception space **(27')** of its structural support **(20),** so that the instillation device can be also used for people whose distance between the nose and the visual axis of the eye may deviate from the range of the normal distance due to anatomy.

**FIG.12** is a perspective view of locking screw **(31)** and according to FIG.11 it passes through the regulatory holes **(30)** of the lateral walls of the modified reception space **(27')** and through the corresponding holes **(30')** of one of the flat projections **(11)** or **(11')** of the reclosable container **(10)** and serves for the stabilization and final immobilization of the reclosable container **(10)** within the modified reception space **(27')** in the desired position for the proper instillation of the eye drops.

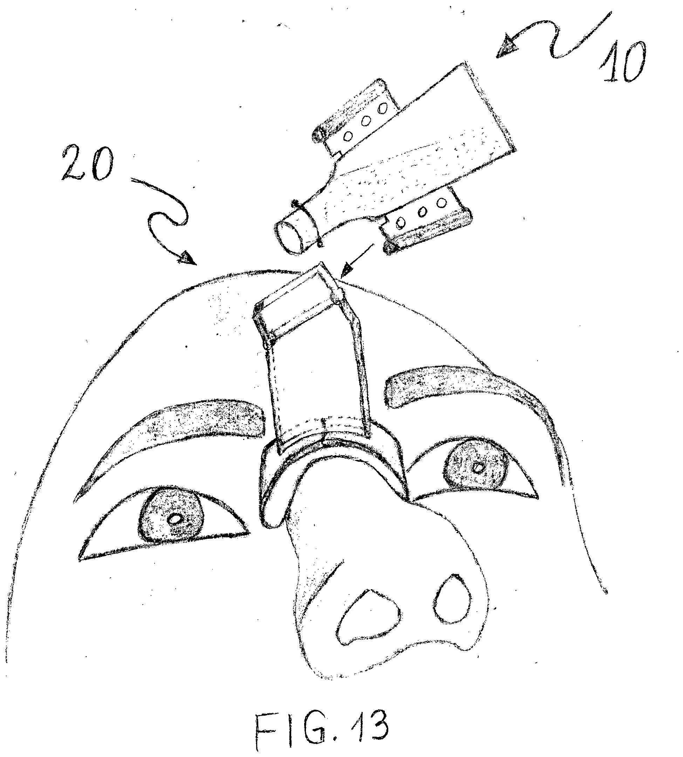

**FIG.13** illustrates the general image of the innovative device for the safe and easy instillation of eye drops, the reclosable container **(10)** and its structural support **(20),** which is adjusted to the upper nasal part of the patient.

## Claims

1. The device for the safe and easy instillation of eye drops is **characterized by** the fact that, it consists of two independent parts, namely : a) the reclosable container **(10)** showing on the walls of its body the flats projections **(11,11'),** the notches **(111,111'),** the holes **(30'),** the cylindricals protruberances **(12, 12'),** which container is secured with the safety cap **(15),** consisting of a thin Π shaped plastic sheet **(21),** a head **(22)** with an cylindrical casing **(18),** having on its inner surface a cone **(14)** which does not protrude from it, one annular groove **(13')** which is firmly engaged with the annular projection **(13)** of the reclosable container's **(10)** neck and b) its structural support **(20)** consisting of the nasal support base **(23)** which comes into extensive contact with the nose of the wearer, shaped like a saddle, which has on its inner surface a lining of a soft material **(29),** where the nasal support base **(23)** continues supporting in a vertical position the boom arm **(24)** ending in either the reception space **(27)** which includes the recessed slot **(25),** the lateral walls **(26),** the locking projection **(28'),** the terminal closed side **(28),** either with the modified reception space **(27')** which in turn is comprised of the recessed slot **(25),** the lateral walls **(26),** the regulatory holes **(30)** corresponding with the holes **(30')** and finally both in the reception space **(27)** and in the modified reception space **(27')** the reclosable container **(10)** is inserted in a tight-fitting, adjustable, inclined direction in relation to the horizontal level creating a safe distance from the surface of the eye.

2. The device for the safe and easy instillation of eye drops in accordance with claim 1, is **characterized by** the fact that, the reclosable container **(10)** presents on the walls of its body bilaterally two flats projections **(11,11')** rectangular-shaped, each of which has a notch **(111, 111'),** holes (**30'**), cylindrical protruberances **(12, 12')** and on the outer wall of its neck between the spout **(16)** and the body of the container must present one annular projection **(13)** and the sealing points **(19)** of the safety cap **(15),** which cap consists of one head **(22)** with cylindrical casing **(18),** having on its inner surface a cone **(14),** one annular groove **(13'),** the external spout **(16')** and on its outer side one thin Π shaped plastic sheet **(21)** bearing on both sides the linear low-height non-slip protuberances **(17)** and finally the safety cap **(15)** itself is fixed and a liquid-tight seal is ensured after its first opening when the annular groove **(13')** firmly interacts with the annular projection **(13)** of the reclosable container **(10)**

3. The device for the safe and easy instillation of eye drops in accordance with claims 1 and 2 , is **characterized by** the fact that, the safety cap variant **(15')** is manufactured identical to the one mentioned in claim 2 safety cap **(15),** that is, it is a variation of this, with the only difference that, the thin protruding wing **(21')** extends only to the extent of the head **(22)** and bears the linear low-height non-slip protuberances **(17)**

4. The device for the safe and easy instillation of eye drops in accordance with claim 1, is **characterized by** the fact that the structural support **(20)** consists of one nasal support base **(23),** bearing on its inner surface a lining of a soft material **(29),** preferably silicone, one vertical boom arm **(24)** and the reception space **(27),** formed by the recessed slot **(25),** the lateral walls **(26),** one locking projection **(28')** and one terminal closed side **(28),** to create a tight fitting during the insertion of the reclosable container **(10)** in the reception space **(27),** which is inclined with respect to the horizontal level and finnaly these parts functioning throughout this innovative device as a coupling connection type male / female system

5. The device for the safe and easy instillation of eye drops in accordance with claims 1 and 4, is **characterized by** the fact that the modified reception space **(27')** is identical to the one referred in claim 4, corresponding to the reception space **(27),** with the only difference that it consists only of a recessed slot **(25)** and the lateral walls **(26)** which bear in addition the regulatory holes **(30),** corresponding to the holes (**30**') of one of the flat projections **(11)** or **(11')** of the reclosable container **(10),** which is fixed in a inclined position relative to the horizontal level, through a locking screw **(31)** or by another more appropriate form of safety.

6. The device for the safe and easy instillation of eye drops in accordance with claim 1, is **characterized by** the fact that the structural support **(20)** and the reclosable container **(10)** bear markings of the same color, applied either during their manufacture or by the subsequent addition with a permanent pen to their materials of manufacturing, for the easier distinction of different pharmaceutical contents of the reclosable containers **(10)**

7. The device for the safe and easy instillation of eye drops in accordance with claim 1, is **characterized by** the fact that the casing of the reclosable container **(10)** with the safety cap **(15)** are made from non-toxic material, plastic or another suitable material, which has thermal insulation properties to protect the contents of the container from higher than the specified temperatures and environmentally friendly properties, for the protection of the environment from the materials of the empty discarded container and with the possibility of permanent printing of the pharmaceutical information e.g. name of the pharmaceutical solution, expiration date, etc.

8. The device for the safe and easy instillation of eye drops in accordance with claim 1, is **characterized by** the fact that, the structural
support **(20)** is made of a durable, rigid, easy to be cleaned, lightweight, non-toxic and environmentally friendly material, e.g. plastic or another suitable material and in this way the device becomes easy to be transported and easy to be used, due to its light weight and small dimensions as well as its ergonomic design.

9. The use of the structural support **(20)** of the device for the safe and easy instillation of eye drops in accordance with claim 1, is **characterized by** the fact that, the structural support **(20)** itself is reusable

10. The use of the structural support **(20)** of the device for the safe and easy instillation of eye drops in accordance with claim 1, is **characterized by** the fact that, the structural support **(20)** itself is used alternately in both eyes of the patient according to the medical treatment instructions.
